# EUROPEAN PATENT SPECIFICATION

(11) **EP 1 551 277 B1**
(45) Date of publication and mention of the grant of the patent: **08.08.2007**
(21) Application number: 03751231.6
(22) Date of filing: 03.10.2003
(51) Int. Cl.: A61M 16/04

(54) **ENDOTRACHEAL TUBE WITH AN IMAGING SENSOR**
ENDOTRACHEALTUBUS MIT EINEM BILAUFNAHMESENSOR
TUBE ENDOTRACHÉAL COMPRENANT UN CAPTEUR D'IMAGE

(30) Priority: 03.10.2002 US 415550 P
(43) Date of publication of application: 13.07.2005
(60) Divisional application: 07100474.1
(73) Proprietor: Etview Ltd., 20179 Misgav (IL)
(72) Inventor: GAVRIELY, Oren, 34618 Haifa (IL)
(74) Representative: Messulam, Alec Moses
(86) International application number: PCT/IL2003/000797
(87) International publication number: WO 2004/030527

(56) References cited:
- EP-A- 0 712 601
- WO-A-01/54565
- WO-A-94/28784
- WO-A-02/085194
- US-A- 6 164 277
- US-A1- 2002 072 680
- US-B1- 6 266 547

## Description

### TECHNICAL FIELD OF THE INVENTION

The present invention relates generally to a medical means of monitoring critically ill and anesthetized patients including monitoring ventilated patients. More specifically, the invention is a device for monitoring patient's organs and cavities.

### BACKGROUND OF THE INVENTION

Insertion of tubes into patient's body organs, cavities and tracts is a common procedure in emergency and critical care medicine. An endotracheal tube may be inserted into the trachea of a patient who is in acute respiratory failure or is undergoing general anesthesia. The endotracheal tube, a typical example of which is shown in YS 6,164,277, must be placed quickly and accurately and positioned with its tip in the mid portion of the patient's trachea to prevent accidental slipping and to provide proper seal and ventilation of both lungs. Similarly, a naso - gastric tube is commonly inserted through the nose or mouth into the stomach of patients who need artificial feeding or evacuation of the content of the stomach. Another tube that is frequently inserted into a body cavity during emergency treatment is the urinary catheter. This catheter is threaded through the urethra into the urinary bladder. The correct placement of these tubes and catheters throughout their use is critically important.

Many patients who are critically ill or undergoing general anesthesia require artificial ventilation. For over 40 years the most common method of providing artificial ventilation has been by pumping compressed air into the patient's lungs through an endotracheal tube. This tube is inserted through the patient's mouth or nose and passed between the vocal cords into the trachea. Alternatively, a :tube may be inserted into the trachea through a tracheotomy surgical incision.

For oral intubation the operator usually uses a laryngoscope, which consists of a handle and a blade. The operator inserts the blade into the patient's mouth and advances it until its tip lies in the pharynx beyond the root of the tongue. The handle is then used to manipulate the blade and push the tongue out of the Nay until the epiglottis and the vocal folds can be seen. The tip of the endotracheal tube can then be aimed and pushed between the vocal folds into the trachea. This method of insertion is used in the majority of intubations, but requires skill, training and experience and is only performed by specialized physicians and licensed paramedics.

An alternative method that is often used when difficult intubation is anticipated is over a fiber optic bronchoscope. First the bronchoscope is connected to a light source to provide the needed illumination of the field facing its tip. The shaft of the bronchoscope is then inserted through the endotracheal tube and moved in as far as possible. The tip of bronchoscope is then inserted into the patient's airway and advanced under visualization through the bronchoscope's eyepiece or a video display in between the vocal folds into the trachea. The endotracheal tube can now be pushed down the bronchoscope shaft and moved between the vocal folds into the trachea. The endotracheal tube can now be secured and the bronchoscope removed to free up the lumen of the endotracheal tube. While the bronchoscopic method is safer than with the laryngoscope, the equipment needed is expensive, delicate and more cumbersome and is seldom found in the field or on emergency medical vehicles.

Securing the endotracheal tube and preventing its inadvertent movement during use is critical to the prevention of dire accidents. Inflating a cuff that surrounds the tube near its tip occludes the space between the outer wall of the tube and the inner wall of the trachea to provide an airtight seal. The cuff is connected to the external end of the endotracheal tube through a thin channel in the tube's wall. The channel is connected to a one-way valve through which air can be injected to inflate the cuff to the desired pressure and volume. The cuff is also helpful in securing the tube in place, but additional fasteners are usually applied around the head to prevent the tube from slipping in or dislodging.

Once the tube has been inserted, it is mandatory to verify its correct position. Accidental insertion of the tube into the esophagus or placing it too deep inside the airways, so that its tip is lodged in one of the main stem bronchi instead of in the trachea may lead to catastrophic consequences and asphyxiation. Many methods are available to verify the endotracheal tube placement. Auscultation of both sides of the chest is usually done to verify symmetric air entry into both lungs. A chest x-ray is another well-tested method of verifying the tube placement. The x-ray picture reveals the relationships between the endotracheal tube tip and the tracheal first bifurcation (carina). X-ray pictures may be and should be taken whenever an endotracheal tube is placed or repositioned. Additionally, the tube placement may be verified through a fiber optic bronchoscope, by a suction bulb, or through sending and receiving an acoustic signal. These methods are used to verify the initial placement of the endotracheal tube. There are no currently available means for continuous monitoring of the actual placement of the tube.

The advantages of fiber optic visualization were combined with the simple design of the laryngoscope as disclosed by several patents and scientific papers. Additionally, the use of visualization stylets which include means for seeing the airways during the insertion of an endotracheal tube have been described. However, there are no known methods for incorporating the visualization means permanently into the anterior face of the endotracheal tube so that visualization of the airways can be accomplished during the insertion and continuously thereafter.

Endoscopes with imaging sensors in their anterior surface for insertion into the gastro-intestinal tract are known from WO94/28784.

### SUMMARY OF THE INVENTION

The present invention provides a multifunction endotracheal tube as hereinafter set forth in Claim 1 of the appended claims.

### BRIEF DESCRIPTION OF THE DRAWINGS

Fig. 1 is a schematic isometric scheme of the tube of the invention incorporating three types of conduits;
Fig. 2 is a schematic isometric description of a portion of the anterior face of the tube of the invention into which a miniature video camera is incorporated;
Fig. 3 is a schematic description of the items commuting along the tube of the invention, related to the performance of inspection tasks.

### DETAILED DESCRIPTION OF THE PRESENT INVENTION

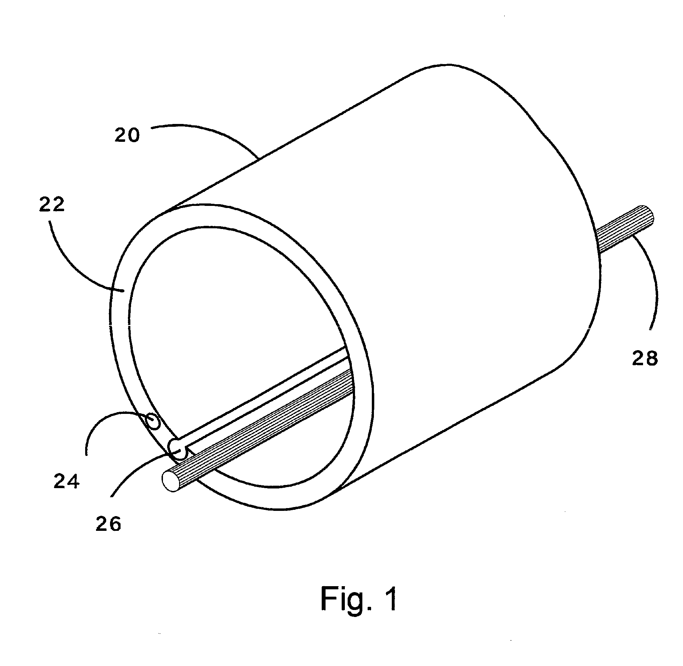

In the illustrated embodiment of the invention, a multifunctional inspection tube is provided for collecting information about internal cavities and spaces in the body of a patient or an animal in association with the insertion of an inspection tube in the body. The multifunctional inspection tube is a modified endotracheal tube. In accordance with the present invention the tube is equipped with means to examine both the positioning of the inspection tube with respect to body organs and the functional aspects of the body during and after the insertion. Thus, the tube of the invention may be used to perform not only customary medical treatment tasks of conveying gasses to and from the penetrated organs, but also inspection tasks that examine the reaction to such treatment and otherwise the condition of the penetrated organs. The multifunctional inspection tube incorporates a means of receiving signals relating to the condition of the penetrated organs such as visual and audio signals by employing suitable sensors incorporated at or near the anterior face of a tube. The signals produced by the sensors are transmitted via wires or communication fibers running along the length of the tube to a connector or a wireless transmitter located at the posterior portion of the tube near its standard connector to the ventilation source. The signals are received by a receiver containing a suitable signal conditioning means for subsequent processing, display, recording and/or monitoring. The structural concept of the invention is better explained with reference to Fig. 1. A portion 20 of a multifunctional inspection tube of the invention is shown, including an anterior face 22. Channels and a conductor are associated with the wall of the tube. A totally embedded channel 24 runs along the length of the inspection tube within its wall, alongside an open recessed channel 26. A conducting element 28 runs along the length of the tube without being embedded in the wall of the tube, rather it is attached to the wall of the tube and occupies a space in the lumen of the tube. The conduit may be partially embedded in the tube or it may be inserted within a recess or it may be threaded within a totally embedded channel without being attached to the tube. Even in embodiments in which the lumen contains a conducting element attached to the wall as described above, the lumen of the tube is still largely free for transferring gasses in both directions. With this respect, embodiment in which the channels and conducting elements embedded or wholly inserted in the wall may be preferable.

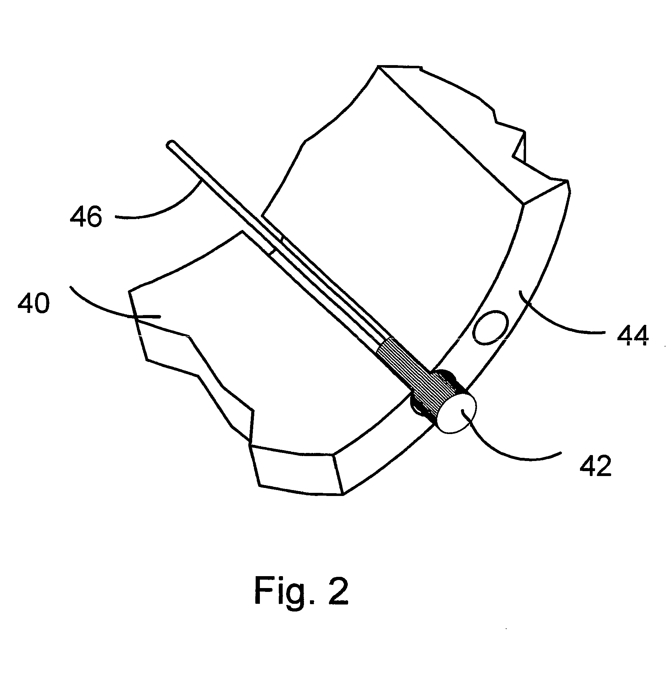

A typical feature of the multifunctional inspection tube is the acquisition of internal images of the body. For acquiring the images, an image sensor may be employed, such as a miniature electronic camera employing a CCD or a CMOS chip incorporated in the anterior face of the inspection tube. In one embodiment, the camera is incorporated in a recess in the wall of the tube as described in Fig. 2 to which reference is now made. A portion of an inspection tube is shown, within the inner side 40 of which, a camera 42 is inserted in a recessed channel, protruding from the anterior face 44 of the tube. The signal of the camera is transmitted by a conducting element 46, typically a copper wire or an optical fiber. The camera's lens is facing away from the tube. The signals arriving from the camera are subsequently fed to a receiver and may be subsequently displayed on a screen, which may be a stand-alone mini-screen, an ordinary video screen, or a portion of the display screen ordinarily used to monitor the physiological parameters and well being of the patient. In some embodiments of the invention, a fiber optical element running along the length of the tube is used to convey light to illuminate the field of view ahead of the anterior face of the inspection tube. Alternatively, a light source may be associated with the proximal face of the tube. Examples for light sources are miniature halogen lamps, light emitting diodes (LED), lasers, or any other kind of light-emitting source of suitable size. An alternative method of illumination is by constructing the inspection tube made of light - conducting material. In some embodiments of the invention, means for keeping the lens of the camera and /or other sensors clean and clear are employed. The airways of an ill patient are often filled with secretions that may be thick and viscous. Thus, it is quite possible that the secretions may lodge on the lens and obscure its field of view, or on other sensors thereby modifying their responsiveness. To overcome such an obstacle, a constant or intermittent flow of air or physiological fluid is pumped through a channel in the tube's wall, whereby the outlet of the channel is aimed directly over and around the lens or the sensor's active surface. This flow may be generated by a simple flow source or by a device that is triggered to emit flow upon command from a human care giver, a timer or a software program that monitors the signal and determines when clearing action is required.

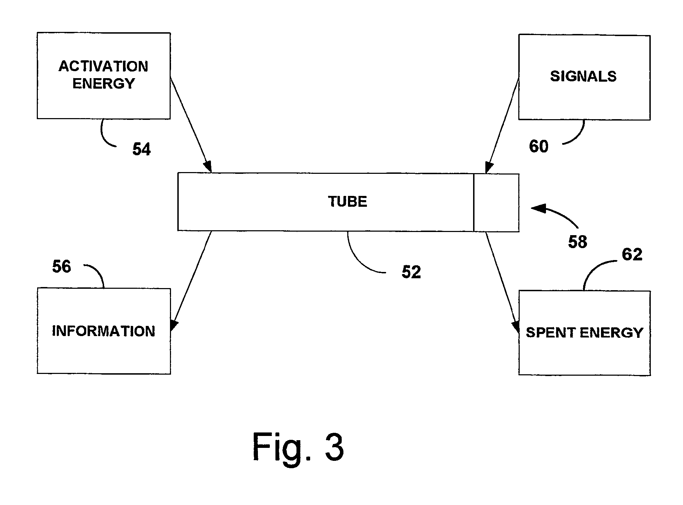

In general, the inspection tube is used as bi - directional conveying platform for various elements required for the fulfillment of its inspection tasks. This is described schematically in Fig. 3 to which reference is now made. Tube 52 receives activation energy 54 of one or several types on its rear end, and downloads information 56, raw or processed at the same end. At the anterior and 58, the tube receives signals 60 of one or several types, and spends energy 62 as will be elaborated later on.

In some embodiments of the invention, a microphone is employed in the tube. Such a microphone can be incorporated in the wall of the tube. Such a microphone receives acoustic signals from at least the vicinity of the tube's anterior, and transfers the signals, raw or processed to the rear of the tube for further downloading and processing.

A plurality of sensors can be effectively employed in the anterior face of the tube of the invention, the non-exhaustive list includes cameras, video cameras, microphones, pressure transducers and thermal sensors. Gas sensors, for example sensors for particular gasses such as oxygen and carbon dioxide may also be employed. The energy required to activate such sensors is supplied by conduits of energy such as electric wires incorporated in the tube. In addition, auxiliary energy can be supplied to the vicinity of the anterior face of the tube for the purpose of cleaning and clearing the sensors active facets by flushing them with cleaning media such as gases, humidified air or oxygen, or liquids, typically a physiological solution, through channels in the wall of the tube. Liquids and or gases for flushing are energized and conducted typically via a totally embedded channel. The inspection tube of the invention may be used alone or in combination with other catheters and tubes that are ordinarily inserted into lung airways. The present technology may be applied in artificial ventilation of the lung.

The sensors of the tube transmit one or more signal types, which are either preprocessed in the sensor for example on the CCD chip, or may be sent raw, to be further processed by analog or digital circuits to yield information relating to the status of the organ or body cavity inspected. The receiving and or processing devices such as, monitors, displays, storage means, analyzers, DSP processors, computers and generators of alarm signals are typically connected by one or a plurality of connectors to the tube. The tube of the invention may be used for insertion through orifices such as the nose or mouth.

The transmission of raw or preprocessed signals is affected through conductors along the tube such as wires or optical fibers, which connect to a connector at the rear of the tube. A wireless transmitter or transceiver may be applied anywhere suitable on the tube, typically at the rear, for communicating with a console containing a receiver and processor and or a control module.

The inspection tube of the invention may also be used to detect changes in indications of vital functions of a patient. Accordingly, image and acoustic signal are being detected, processed and compared to a reference base picture or sound structure. An alarm is set as soon as certain changes in the indication pass a predetermined threshold. For example, the accumulation of secretions, or development of excessive or diminished lung noises are abnormal.

## Claims

1. A multifunctional endotracheal tube (52) having a substantially free lumen for conveying various elements and gases into and out of the lungs and air passages penetrated by said tube (52), the tube being **characterized by**:
an imaging sensor (42) incorporated in the anterior face (22,44) of said tube,
at least one conduit of energy (54) to activate said imaging sensor (42) is associated with the wall of said tube (52);
at least one conducting element (28) for transmitting image signals of said imaging sensor to the rear end of said tube, and
at least one receiver for said image signals.

2. A multifunction tube (52) as claimed in claim 1 and wherein the wall of said tube (52) comprises a channel (24,26).

3. A multifunction tube (52) as claimed in claim 2, and wherein the wall (20) of said tube comprises a totally embedded channel (24).

4. A multifunction tube as claimed in claim 2, and wherein the wall of said tube comprises a recessed channel (26).

5. A multifunction tube as claimed in claim 1 and wherein a lighting element is associated with said anterior face of said tube.

6. A multifunction tube as claimed in claim 5 and wherein said lighting element is an optical fiber running along the wall said tube.

7. A multifunction tube as claimed in claim 5 and wherein said lighting element is a light emitting source.

8. A multifunctional tube (52) as claimed in claim 1 further comprising cleaning means for keeping said imaging sensor clear.

9. A multifunctional tube (52) as claimed in claim 8, wherein said cleaning means are triggered by at least one of a software or a timer.

10. A multifunction tube (52) as claimed in any preceding claim, further including a microphone for receiving acoustic signals from the vicinity of the anterior of the tube.

11. A system comprising a multifunctional tube (52) as claimed in any preceding claim, in combination with at least one circuit for processing signals provided by said sensor (42) so as to yield information relating to the status of the lungs and air passages being inspected by said tube (52).

12. A system as claimed in claim 11, further comprising means for detecting changes in indication of vital functions comprising means for processing and comparing the image signals to a reference base picture, an alarm to be set as soon as certain changes in the indication pass a predetermined threshold.

13. A system as claimed in claim 11 or 12, wherein said at least one circuit communicates with said sensor through wired or wireless communication.

14. A multifunctional tube as claimed in any of claims 1 to 10, wherein the sensor (42) is adapted to send signals to display and monitoring devices so as to yield information relating to the status of the lungs and air passages being inspected by said tube (52).

## Patentansprüche

1. Ein vielseitiger Endotrachealschlauch (52) der ein beträchtliches freies Lumen zum Fördern von verschiedenen Elementen und Gasen zu und von den Lungen und den Atemwegen hat, welche von dem besagten Schlauch (52) penetriert werden. Dieser Schlauch wird charakterisiert durch:
einen Bildsensor (42), welcher mit vorhergehende Oberfläche (22,42) des besagten Schlauches verbunden ist
mit mindestens einer Energiezufuhr (54), zum Aktivieren des Bildsensors (42) in Verbindung mit der Wand des besagten Schlauches (52)
mit mindestens einem leitenden Element (28) zum Übertragen der Bildsignale des besagten Bildsensors zum hinteren Ende des besagten Schlauches
mit mindestens einem Empfänger für die besagten Bildsignale.

2. Ein vielseitiger Endotrachealschlauch (52), wie im Anspruch 1 beansprucht und worin die besagte Wand des besagten Schlauches (52) aus einem Kanal besteht (24, 26).

3. Ein vielseitiger Schlauch (52), wie im Anspruch 2 beansprucht und worin die besagte Wand (20) des besagten Schlauches aus einem vollständig eingebetteten Kanal (24) besteht.

4. Ein vielseitiger Schlauch, wie im Anspruch 2 beansprucht und worin die Wand des besagten Schlauches aus einem vertieften Kanal (26) besteht.

5. Ein vielseitiger Schlauch, wie im Anspruch 1 beansprucht und worin ein Beleuchtungselement mit der besagten Oberfläche des besagten Schlauches verbunden ist.

6. Ein vielseitiger Schlauch wie im Anspruch 5 beansprucht und worin das besagte Beleuchtungselement ein Lichtleiter ist, der entlang der Wand des besagten Schlauches verläuft.

7. Ein vielseitiger Schlauch wie im Anspruch 5 beansprucht und worin besagtes Beleuchtungselement eine lichtspendende Quelle ist.

8. Ein vielseitiger Schlauch (52), wie im Anspruch 1 beansprucht und der ferner aus einer Reinigungsvorrichtung besteht, um den besagten Bildsensor sauber zu halten.

9. Ein vielseitiger Schlauch (52), wie im Anspruch 8 beansprucht und worin die besagte Reinigungsvorrichtung durch eine Software des Timers ausgelöst wird.

10. Ein vielseitiger Schlauch (52), wie in den vorgenannten Ansprüchen beansprucht, der ferner ein Mikrofon zum Empfangen der akustischen Signale von der Umgebung der Oberfläche des Schlauches hat.

11. Ein Gerät bestehend aus einem vielseitigem Schlauch (52) wie in den vorgenannten Ansprüchen beansprucht in Kombination mit mindestens einem Schaltkreis zum Verarbeiten der Signale, die von dem Bildsensor (42), zum Auswerten der Informationen in Bezug auf die Lungen und der Atemwege übertragen werden, die von dem besagten Schlauch (52) untersucht werden.

12. Ein Gerät wie in Anspruch 11 beansprucht, das ferner über Vorrichtungen verfügt, um Anzeichen einer Änderungen der Vitalfunktionen zu erkennen und über Vorrichtungen zum Verarbeiten und Vergleichen der Bildsignale mit einem Referenzbild und einem Alarm, der ausgelöst wird, wenn bestimmte Änderungen in der Indikation eine vorbestimmte Schwelle übersteigen.

13. Ein Gerät wie in Anspruch 11 und 12 beansprucht, worin mindestens ein Schaltkreis mit dem besagten Sensor über Kabel und drahtlose Kommunikation kommuniziert.

14. Ein vielseitiger Schlauch (52), wie im Anspruch 1-10 beansprucht, worin der Sensor (42) angepasst ist, um Signale an Anzeige- und Überwachungsgeräte zu senden, um Informationen in Bezug auf den Status der Lungen und der Atemwege, die von dem besagten Schlauch (52) untersucht werden, auszuwerten.

## Revendications

1. Un tube endotrachéal multifonctionnel (52) ayant un important espace libre permettant de transporter divers éléments et gaz dans et hors des poumons et des voies aériennes, lesquels sont pénétrés par le dit tube (52), le tube **se caractérisant par** :
Un capteur d'imagerie (42) incorporé à la face interne (22, 44) du dit tube,
Au moins un conduit d'énergie (54) pour activer le capteur d'imagerie (42) susmentionné est associé à la paroi du tube (52) susmentionné ;
Au moins un élément de conduction (28) pour la transmission de signaux d'images du dit capteur à l'extrémité arrière du tube susmentionné ;
Au moins un récepteur de signaux d'images ci-dessus indiqués.

2. Un tube multifonction (52) tel que stipulé dans la déclaration 2, et dans lequel la paroi du dit tube (52) comporte un canal (24, 26).

3. Un tube multifonction (52) tel que stipulé dans la déclaration 2, et dans lequel la paroi (20) du dit tube comporte un canal intégralement intégré (24).

4. Un tube multifonction tel que stipulé dans la déclaration 2, et dans lequel la paroi du dit tube comporte un canal encastré (26).

5. Un tube multifonction tel que stipulé dans la déclaration 1, et dans lequel un élément d'éclairage est associé à la face interne du dit tube.

6. Un tube multifonction, tel que stipulé dans la déclaration 5, et dans lequel un élément d'éclairage est une fibre optique courant le long de la paroi du dit tube.

7. Un tube multifonction tel que stipulé dans la déclaration 5, et dans lequel l'élément d'éclairage susmentionné est une source d'émission de lumière.

8. Un tube multifonction (52), tel que stipulé dans la déclaration 1, comprenant en outre des moyens de nettoyage pour maintenir le capteur d'imagerie propre.

9. Un tube multifonction (52) tel que stipulé dans la déclaration 8, et dans lequel les moyens de nettoyage sont déclenchés au moins un logiciel ou un compteur.

10. Un tube multifonction (52), tel que stipulé dans les déclarations précédentes, incluant en plus un microphone pour la réception de signaux acoustiques à proximité de la partie interne du tube.

11. Un système se composant d'un tube multifonctionnel (52), tel que stipulé dans les déclarations précédentes, en combinaison avec au moins un circuit pour le traitement des signaux, lesquels sont fournis par le dit capteur (42), afin de produire des informations relatives à l'état des poumons et des voies aériennes examinés par le tube (52) susmentionné.

12. Un système, tel que stipulé dans la déclaration 11, se composant en plus de moyens permettant de détecter des modifications dans les indications de fonctions vitales, incluant des moyens de traitement et de comparaison de signaux d'images à une image de base de référence, une alarme à régler dès que certaines modifications d'indication dépassent un seuil prédéterminé.

13. Un système, tel que stipulé dans les déclarations 11 ou 12, dans lequel au moins circuit communique avec le capteur susmentionné via une communication câblée ou sans fil.

14. Un tube multifonctionnel, tel que stipulé dans les déclarations 1 à 10, dans lequel le capteur (42) est adapté pour émettre des signaux aux périphériques d'affichage et de surveillance, afin de produire des informations relatives à l'état des poumons et des voies aériennes examiné par le tube (52) susmentionné.
